# EUROPEAN PATENT APPLICATION

(11) **EP 3 620 106 A1**
(43) Date of publication of application: **11.03.2020**
(21) Application number: 18193384.7
(22) Date of filing: 10.09.2018
(51) Int. Cl.: A61B 5/021, A61B 5/00, A61B 5/0295

(54) **DEVICE FOR USE IN MEASURING BLOOD PRESSURE**

(71) Applicant: Koninklijke Philips N.V., 5656 AE Eindhoven (NL)
(72) Inventor: PRESURA, Cristian Nicolae, 5656 AE Eindhoven (NL); AARTS, Ronaldus Maria, 5656 AE Eindhoven (NL)
(74) Representative: de Haan, Poul Erik

(57) **Abstract**

There is provided a device (100) comprising an inner member (102) configured to be worn around a finger (104) of a subject and an outer member (106) positioned in a spatial arrangement with respect to the inner member (102). The device (100) comprises at least one light source (108) arranged on the inner member (102) and/or the outer member (106), which is configured to emit light (112) to illuminate the finger (104). The device (100) comprises at least one light detector (110) arranged on the inner member (102) and/or the outer member (106), which is configured to obtain optical signals from the finger (104). The inner member (102) is movable relative to the outer member (106) to enable the at least one light detector (110) to obtain optical signals for different light paths through the finger (104) for use in measuring blood pressure.

## Description

### FIELD OF THE INVENTION

The disclosure relates to a device and method of operating the device for use in measuring blood pressure.

### BACKGROUND OF THE INVENTION

Blood pressure is considered to be one of the most important physiological parameters for monitoring subjects. This is especially the case for subjects in a critical condition. Blood pressure can, for example, provide a useful indication of the physical or physiological condition of a subject. A variety of techniques exist for measuring blood pressure. The techniques can be divided into two categories, namely invasive techniques and non-invasive techniques. Most invasive techniques are expensive and are generally only used in hospitals (especially in intensive care units) as they require specialists for operation. Therefore, non-invasive techniques can be more practical where blood pressure measurements need to be acquired more frequently by an untrained user.

A common non-invasive technique for measuring the blood pressure of a subject is that which uses light to illuminate a finger of the subject and a detector to obtain an optical signal from the finger for use in measuring blood pressure. The optical signal that is obtained in this technique reflects the blood movement in a vessel, which goes from the center of the body (i.e. the heart) to the extremities of the body (e.g. the fingertips) in a wavelike motion. Photoplethysmography (PPG) and pulse oximetry are example techniques where light is used to estimate the blood flow through the skin of the finger of the subject.

Many devices that are configured to be worn around a finger of a subject have been developed to obtain an optical signal from the finger for use in measuring blood pressure. However, an important problem with respect to sensors that are wearable or part of a wearable apparatus, and especially with respect to sensors that rely on a measurement of light (such as PPG sensors), is ensuring that the finger remains stationary with respect to the light sensor and/or light source. Otherwise, motion artifacts influence the amplitude of the optical signal and this results in increased errors in blood pressure measurements obtained using the optical signal. The motion artifacts are particularly apparent in an optical signal obtained from a finger of a subject.

An example of a device aimed at addressing motion artifacts is described in "Artifact-Resistant Power-Efficient Design of Finger-Ring Plethysmographic Sensors", by Sokwoo Rhee et al., IEEE Transactions on biomedical engineering, Vol. 48, No. 7, July 2001. The disclosed device is in the form of a ring sensor, where an optical sensor unit comprising light emitting diodes (LEDs) and photodiodes is integrated in, or attached directly to, the body of a ring that is configured to be worn around a finger of a subject. The ring sensor includes an inner ring that holds the optical sensor unit and an outer ring surrounding the inner ring that acts as a housing and contains other components (e.g. a central processing unit, signal processing unit, battery, and radio frequency transmitter).

The inner ring and outer ring are mechanically decoupled from each other, such that external forces are borne by the outer ring and not directly transmitted to the inner ring where the optical sensor is located. However, while a device having this ring arrangement can reduce motion artifacts in an optical signal obtained from a finger of a subject, the device is not optimized for measuring blood pressure from the finger. In particular, the location of the device on the finger of the subject can have an impact on the usefulness of the optical signal in measuring blood pressure and thus on the reliability of blood pressure measurements acquired using the device.

### SUMMARY OF THE INVENTION

As noted above, a limitation associated with existing devices for use in measuring blood pressure is that, while motion artifacts can be reduced, the location of an existing device on a finger of a subject can have an impact on the reliability of blood pressure measurements acquired using the device. It would thus be valuable to provide an improved device aimed at addressing the limitation associated with existing devices.

Therefore, according to a first aspect, there is provided a device for use in measuring blood pressure. The device comprises an inner member configured to be worn around a finger of a subject and an outer member positioned in a spatial arrangement with respect to the inner member. The device also comprises at least one light source arranged on at least one of the inner member and the outer member. The at least one light source is configured to emit light to illuminate the finger of the subject. The device also comprises at least one light detector arranged on at least one of the inner member and the outer member. The at least one light detector is configured to obtain optical signals from the finger of the subject. The inner member is movable relative to the outer member to enable the at least one light detector to obtain optical signals for different light paths through the finger of the subject for use in measuring blood pressure.

In some embodiments, the inner member may be movable relative to the outer member by being any one or more of rotatably movable relative to the outer member and axially movable relative to the outer member.

In some embodiments, the outer member may be positioned in a spatial arrangement with respect to the inner member by being any one or more of positioned around the inner member and displaced along an axis from the inner member.

In some embodiments, the outer member may be mechanically independent of the inner member. In some embodiments, the outer member may be separated from the inner member by a fluid chamber. In some embodiments, the fluid chamber may be configured to hold a pressurized fluid.

In some embodiments, the inner member may be flexible and/or the outer member may be rigid.

In some embodiments, the inner member and the outer member may be arranged such that, in use, only the inner member is in contact with the finger of the subject.

In some embodiments, the inner member may comprise a portion through which any one or more of at least one light source arranged on the outer member is configured to emit light to illuminate the finger of the subject and at least one light detector arranged on the outer member is configured to obtain optical signals from the finger of the subject.

In some embodiments, a distance between the at least one light source and the at least one light detector may be variable.

According to a second aspect, there is provided a system for use in measuring blood pressure. The system comprises the device described earlier. The system may also comprise a processor configured to process the obtained optical signals for the different light paths through the finger of the subject to select a position for the inner member relative to the outer member for use in measuring a blood pressure of the subject.

In some embodiments, the processor may be configured to select the position for the inner member relative to the outer member for use in measuring the blood pressure of the subject based on an amplitude of the obtained optical signals for the different light paths through the finger of the subject.

According to a third aspect, there is provided a method of operating a device as described earlier. The method comprises emitting the light to illuminate the of the subject, moving the inner member relative to the outer member and obtaining, from the finger of the subject, the optical signals for the different light paths through the finger of the subject for use in measuring blood pressure.

In some embodiments, the method can comprise processing the obtained optical signals for the different light paths through the finger of the subject to select a position for the inner member relative to the outer member for use in measuring a blood pressure of the subject.

According to a fourth aspect, there is provided a computer program product comprising a computer readable medium, the computer readable medium having computer readable code embodied therein, the computer readable code being configured such that, on execution by a suitable computer or processor, the computer or processor is caused to perform the method described above.

According to the aspects and embodiments described above, the limitations of existing techniques are addressed. In particular, the above-described aspects and embodiments provide a device where the inner member is movable relative to the outer member to enable at least one light detector arranged on the inner member and/or the outer member to obtain optical signals for different light paths through the finger of the subject for use in measuring blood pressure. In this way, it is possible move the inner member relative to the outer member until an optimum arrangement of the inner member with respect to the outer member is reached, whereby the optical signals obtained in that optimum arrangement provide optimum blood pressure measurements. This is based on the insight that some locations on the finger of the subject provide more reliable blood pressure measurements than other locations on the finger of the subject. In this way, the reliability of blood pressure measurements acquired from optical signals can be improved.

The limitations associated with the existing devices discussed earlier are therefore addressed by way of the above-described aspects and embodiments.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a simplified schematic illustration of a device according to an example embodiment;
Fig. 2 is a simplified schematic illustration of a device according to another example embodiment;
Fig. 3 is a simplified schematic illustration of a device according to another example embodiment;
Fig. 4(a) is a simplified schematic illustration of a device according to another example embodiment;
Fig. 4(b) is a simplified schematic illustration of a device according to another example embodiment;
Fig. 5 is a simplified schematic illustration of a device according to another example embodiment; and
Fig. 6 is a flow chart illustrating a method of operating a device according to an embodiment.

### DETAILED DESCRIPTION OF EMBODIMENTS

There is provided herein device for use in measuring blood pressure. The device can be configured to be worn on a finger of a subject. The subject can be any type of subject, such as a patient or any other subject. The device can be configured for any purpose. For example, the device can be a medical device according to some embodiments or a non-medical device according to other embodiments. The device can be for use by a trained user, such as a medical professional (e.g. a doctor, a nurse, or any other medical professional) or an untrained user (e.g. the subject themselves, a care giver, a family member, or any other untrained user). The device can be for use in a medical environment (e.g. a hospital, a surgery, or any other medical environment) and/or non-medical environment (e.g. at home, or any other non-medical environment).

Briefly, the device described herein comprises an inner member configured to be worn around a finger of a subject and an outer member positioned in a spatial arrangement with respect to the inner member. The device further comprises at least one light source arranged on (e.g. fixed to) at least one of the inner member and the outer member. The at least one light source is configured to emit light to illuminate the finger of the subject. The device also comprises at least one light detector arranged on (e.g. fixed to) at least one of the inner member and the outer member. The at least one light detector is configured to obtain optical signals from the finger of the subject in response to the finger illumination. The inner member is movable relative to the outer member to enable the at least one light detector to obtain optical signals for different light paths through the finger of the subject for use in measuring blood pressure.

In this way, it is possible to move the inner member relative to the outer member until an optimum arrangement of the inner member with respect to the outer member is reached, whereby the optical signals, caused by the finger illumination, obtained in that optimum arrangement provide optimum blood pressure measurements. For example, an arrangement that provides optimum blood pressure measurements can be an arrangement in which the at least one light sensor is positioned close (or closer) to an artery and/or an arrangement in which the at least one light detector can obtain an optical signal for a light path through an artery in the finger of the subject for use in measuring blood pressure e.g. such that the obtained optical signal can be maximized. In this way, the reliability of blood pressure measurements acquired from obtained optical signal can be improved.

The inner member described herein may be movable relative to the outer member in any way. In some embodiments, the inner member may be movable and the outer member may be fixed in order for the inner member to be movable relative to the outer member. In other embodiments, the outer member may be movable and the inner member may be fixed in order for the inner member to be movable relative to the outer member. In yet other embodiments, the inner member and the outer member may be movable in order for the inner member to be movable relative to the outer member.

In some embodiments, the inner member described herein may be movable relative to the outer member by being rotatably movable relative to the outer member. Thus, the inner member can rotate and/or the outer member can rotate according to some embodiments. Alternatively or in addition, in some embodiments, the inner member described herein may be movable relative to the outer member by being axially movable (i.e. moveable along an axis or translatable) relative to the outer member. Thus, the inner member can move axially (i.e. along an axis) and/or the outer member can move axially (i.e. along an axis) according to some embodiments. That is, the inner member can be translated and/or the outer member can be translated according to some embodiments.

In some embodiments, a distance between the at least one light source and the at least one light detector described herein can be variable. For example, in some embodiments, the distance between the at least one light source and the at least one light detector described herein can be altered or changed, for example, by mean of relative displacement of the inner and outer members. In some embodiments, the distance between the at least one light source and the at least one light detector described herein can be variable by the at least one light source being arranged on one of the inner member and the at least one light detector being arranged on the outer member or by the at least one light source being arranged on one of the outer member and the at least one light detector being arranged on the inner member. In particular, the movement of the inner member relative to the outer member can allow the distance between the at least one light source and the at least one light detector to be varied.

The inner member and the outer member described herein can each comprise a hollow internal region. For example, in some embodiments, any one or more of the inner member and the outer member described herein may be circular (e.g. ring-shaped) or any other shape having a hollow internal region. The inner member described herein is smaller in size than the outer member. For example, in embodiments where the inner member and the outer member are circular, the inner member has a smaller diameter than the outer member. In effect, the inner member can be sized such that it can fit inside the outer member.

In some embodiments, the outer member described herein may be positioned in a spatial arrangement with respect to the inner member by being positioned around the inner member (in a coaxial relationship) and/or displaced along an axis from the inner member. In some embodiments, for example, the inner member described herein may be positioned inside (or at least partially inside) the outer member. In some embodiments, the inner member and the outer member described herein may be positioned concentrically (i.e. may be concentric or concentrically arranged). In embodiments where the outer member described herein is displaced along an axis from the inner member, the inner member may protrude from the outer member or be positioned adjacent to the outer member. In this way, the inner member is accessible.

In some embodiments, the outer member described herein may be mechanically independent of (decoupled from) the inner member. More specifically, there may be no mechanical connection between the outer member and the inner member according to some embodiments. In embodiments where at least one light source is arranged on the inner member, the at least one light source arranged on the inner member may be electrically connected to the outer member. Similarly, in embodiments where at least one light detector is arranged on the inner member, the at least one light detector arranged on the inner member may be electrically connected to the outer member.

In some embodiments, the outer member described herein may be separated from the inner member by a fluid chamber, such as a liquid and/or gas chamber. The fluid chamber described herein is a chamber suitable to hold (e.g. be filled with) a fluid. According to some embodiments, the chamber described herein may be configured to hold (e.g. be filled with) a pressurized fluid. Thus, pressure can be applied on the finger of the subject by way of the fluid chamber described herein. This applied pressure can block or at least partially block one or more arteries in the finger of the subject. The fluid referred to herein can be any one or more of a liquid and a gas (e.g. air).

In some embodiments, the fluid chamber described herein may be formed from an inner surface of the inner member and an inner surface of the outer member. In other embodiments, the fluid chamber described herein may be formed from an inner surface of the inner member and an inner surface of at least one other member positioned between the inner member and the outer member. In yet other embodiments, the fluid chamber described herein may be formed from an inner surface of the outer member and an inner surface of at least one other member positioned between the inner member and the outer member. In yet other embodiments, the fluid chamber described herein may be a dedicated chamber positioned between the inner member and the outer member. In yet other embodiments, the outer member may itself comprise (or be) the fluid chamber.

In some embodiments, the outer member described herein can be rigid. For example, the outer member described herein may be formed from a rigid material. Alternatively or in addition, the inner member described herein can be flexible. For example, the inner member described herein may be formed from a flexible material. In these embodiments and where the outer member is separated from the inner member by a fluid chamber, the pressure of the fluid within the fluid chamber can be transferred by the flexible inner member to the finger of the subject.

In some embodiments, the inner member and the outer member described herein may be arranged such that, in use, only the inner member is in contact with the (e.g. skin of the) finger of the subject. That is, the inner member and the outer member described herein may be arranged such that, in use, the outer member does not come in contact with the (e.g. skin of the) finger of the subject.

In some embodiments, the inner member described herein can comprise a portion (e.g. an aperture or a transparent portion, such as a window) through which at least one light source is configured to emit light to illuminate the finger of the subject. In other words, in some embodiments, the inner member described herein can comprise a portion (e.g. an aperture or a transparent portion, such as a window) through which at least one light detector is configured to obtain optical signals from the finger of the subject.

For example, in some embodiments, the inner member described herein can comprise a portion (e.g. an aperture or a transparent portion, such as a window) through which at least one light source arranged on the outer member is configured to emit light to illuminate the finger of the subject. In other words, in some embodiments, the inner member described herein can comprise a portion (e.g. an aperture or a transparent portion, such as a window) through which at least one light detector arranged on the inner member is configured to obtain optical signals from the finger of the subject.

Alternatively or in addition, in some embodiments, the inner member described herein can comprise a portion (e.g. an aperture or a transparent portion, such as a window) through which at least one light source arranged on the inner member is configured to emit light to illuminate the finger of the subject. In other words, in some embodiments, the inner member described herein can comprise a portion (e.g. an aperture or a transparent portion, such as a window) through which at least one light detector arranged on the outer member is configured to obtain optical signals from the finger of the subject.

The at least one light source described herein can comprise any light source suitable for emitting light to illuminate the finger of the subject. For example, in some embodiments, the at least one light source may comprise at least one light emitting diode (LED). The at least one light detector described herein can comprise any light detector suitable for obtaining optical signals from the finger of the subject. Thus, the at least one light detector described herein may also be referred to as at least one photodetector. In some embodiments, the at least one light detector may comprise at least one photoplethysmography (PPG) sensor. In these embodiments, the optical signals obtained from the finger of the subject by the at least PPG sensor may be referred to as PPG signals.

There is also provided herein a system for use in measuring blood pressure. The system comprises the device described herein. In some embodiments, the system can also comprise a processor. For example, in some embodiments, the device may comprise or be coupled to the processor. Alternatively or in addition, in other embodiments, the processor may be separate from (e.g. external to or remote from) the device. In embodiments with a processor, the at least one light detector may communicate with the processor wirelessly or via a wired connection. For example, in some embodiments such as any of those described herein, the processor can be configured to acquire the obtained optical signals for the different light paths through the finger of the subject from the at least one light detector wirelessly or via a wired connection. The processor may be one or more processors (such as one or more microprocessors, one or more multi-core processors and/or one or more digital signal processors (DSPs)), one or more processing units, and/or one or more controllers (such as one or more microcontrollers) that may be configured or programmed (e.g. using software or computer program code) to perform the various functions described herein.

According to some embodiments, such as any of those described herein, the processor can be configured to process the obtained optical signals for the different light paths through the finger of the subject to select a position for the inner member relative to the outer member for use in measuring a blood pressure of the subject. In some embodiments, the processor described herein may be configured to select the position for the inner member relative to the outer member for use in measuring the blood pressure of the subject based on an amplitude of the obtained optical signals for the different light paths through the finger of the subject. In this way, an optimum arrangement of the inner member with respect to the outer member can be selected, whereby the optical signals obtained in that optimum arrangement provide optimum blood pressure measurements.

Thus, in effect, the processor described herein can be configured to search for the optimum arrangement for blood pressure measurements. In some embodiments, any of the functionality described herein can be automated or at least partially automated. In some such embodiments, for example, the processor can be configured to automatically search for the optimum arrangement for blood pressure measurements.

In some embodiments, the processor described herein may be configured to control a user interface to render (e.g. output or provide) an output indicative of the optimum arrangement of the inner member with respect to the outer member being reached. For example, the processor described herein may be configured to control a user interface to render (e.g. output or provide) an output indicative of the selected position for the inner member relative to the outer member for use in measuring the blood pressure of the subject being reached. The output can be any type of output, such as a visual output, an audio output, a haptic output, or any other output, or any combination of outputs. In some embodiments, the device described herein may itself comprise the user interface. Alternatively or in addition, the user interface may be separate from (e.g. external to or remote from) the device described herein. For example, the user interface may be part of another device or a stand-alone user interface. The processor described herein may be configured to control the user interface in the manner described herein via a wired or a wireless connection.

In an example of an embodiment where the output is a visual output, the user interface may comprise one or more lights (e.g. one or more light emitting diodes (LEDs)) and the processor described herein can be configured to control the one or more lights to render (e.g. output or provide) the output. For example, the processor described herein may be configured to turn on one or more green lights (e.g. with any red lights turned off) to indicate that the optimum arrangement of (e.g. the selected position for) the inner member with respect to the outer member is reached. Alternatively or in addition, the processor described herein may be configured to turn on one or more red lights (e.g. with any green lights turned off) to indicate that the optimum arrangement of (e.g. the selected position for) the inner member with respect to the outer member is not reached. In some embodiments, the processor described herein may be configured to turn on one or more red lights until the optimum arrangement of (e.g. the selected position for) the inner member with respect to the outer member is reached, at which point the processor described herein may be configured to turn off the one or more red lights and turn on one or more green lights.

In this way, the user (e.g. the subject themselves or another user) of the device described herein can be provided with feedback, such that the user is aware of the moment at which the optimum position is reached for the blood pressure of the subject to be measured. In some embodiments, the processor described herein can be configured to process an optical signal obtained from the finger of the subject when the optimum arrangement of (e.g. the selected position for) the inner member with respect to the outer member is reached to determine a blood pressure measurement for the subject. Any existing techniques for determining a blood pressure measurement for a subject from an optical signal obtained from the subject can be used and a person skilled in the art will be aware of such existing techniques that may be used.

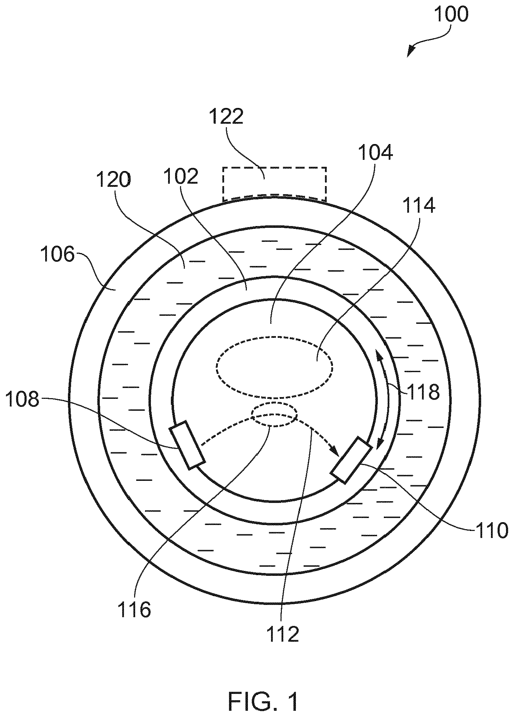

Fig. 1 illustrates an example of the device 100 for use in measuring blood pressure according to an embodiment. As illustrated in Fig. 1, the device 100 comprises an inner member 102 configured to be worn around a finger 104 of a subject. The device 100 also comprises an outer member 106 positioned in a spatial arrangement with respect to the inner member 102. As described earlier, the outer member 106 may be positioned in a spatial arrangement with respect to the inner member 102 by being positioned around the inner member 102 and/or displaced along an axis from the inner member 102.

As mentioned earlier, the device 100 comprises at least one light source 108 arranged on at least one of the inner member 102 and the outer member 106. More specifically, in the illustrated example embodiment of Fig. 1, the at least one light source 108 is arranged on the inner member 102. The at least one light source 108 is configured to emit light 112 to illuminate the finger 104 of the subject. The light 112 is illustrated in Fig. 1 with reference to a bone 114 of the finger 104 of the subject and an artery 116 in the finger 104 of the subject. Although only one light source 108 is illustrated in the example embodiment of Fig. 1, it will be understood that the device 100 may alternatively comprise a plurality of light sources 108.

As mentioned earlier, the device 100 described herein comprises at least one light detector 110 arranged on at least one of the inner member 102 and the outer member 106. In the illustrated example embodiment of Fig. 1, the at least one light detector 110 is arranged on the inner member 102. The at least one light detector 110 is configured to obtain optical signals from the finger 104 of the subject. Although only one light detector 110 is illustrated in the example embodiment of Fig. 1, it will be understood that the device 100 may alternatively comprise a plurality of light detectors 110.

As described earlier, the inner member 102 is movable relative to the outer member 106 to enable the at least one light detector 110 to obtain optical signals for different light paths through the finger 104 of the subject for use in measuring blood. As illustrated by the arrow 118, in some embodiments such as the example illustrated in Fig. 1, the inner member 102 can be movable relative to the outer member 106 by being rotatable relative to the outer member 106. Although an example of the movement of the inner member 102 relative to the outer member 106 has been provided with reference to the example embodiment illustrated in Fig. 1, it will be understood that the inner member 102 can be movable relative to the outer member 106 in alternative or additional ways in other embodiments. For example, in some embodiments, the inner member 102 may alternatively or additionally be axially movable relative to the outer member 106 and/or moveable in any other way.

Thus, it is possible to move the inner member 102 relative to the outer member 106 until an optimum arrangement of the inner member 102 with respect to the outer member 106 is reached, whereby the optical signals obtained in that optimum arrangement provide optimum blood pressure measurements. For example, an arrangement that provides optimum blood pressure measurements can be an arrangement in which the at least one light detector 110 can obtain an optical signal for a light path through the artery 116 in the finger 104 of the subject for use in measuring blood pressure. In this way, the reliability of blood pressure measurements acquired from obtained optical signal can be improved.

As illustrated in Fig. 1 and as described earlier, in some embodiments, the outer member 106 can be separated from the inner member 102 by a fluid (e.g. liquid or gas) chamber 120.

As also illustrated in Fig. 1, in some embodiments, the device 100 may comprise or be coupled to a processor 122 such as that described earlier. However, it will be understood that in other embodiments with a processor 122, the processor 122 may be separate from (e.g. external to or remote from) the device 100. In embodiments with a processor 122, as described earlier, the at least one light detector 110 may communicate with the processor 122 wirelessly or via a wired connection (not illustrated in Fig. 1).

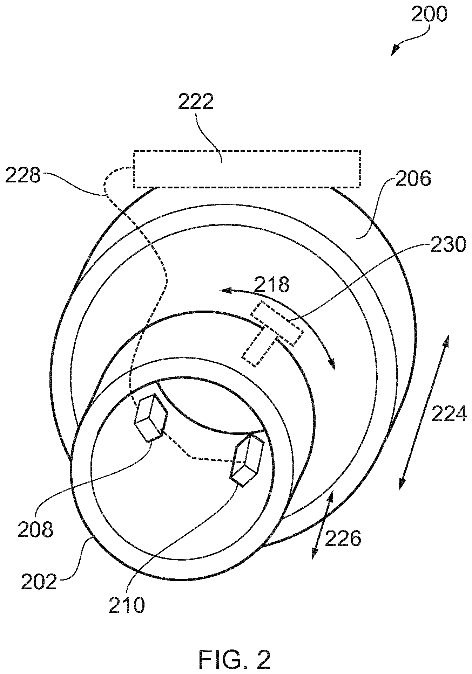

Fig. 2 illustrates an example of the device 200 for use in measuring blood pressure according to another embodiment. As illustrated in Fig. 2, the device 200 comprises an inner member 202 configured to be worn around a finger of a subject (not illustrated in Fig. 2). The device 200 also comprises an outer member 206 positioned in a spatial arrangement with respect to the inner member 202. As described earlier, the outer member 206 may be positioned in a spatial arrangement with respect to the inner member 202 by being positioned around the inner member 202 and/or displaced along an axis from the inner member 202. In the example embodiment illustrated in Fig. 2, the outer member 206 is positioned in a spatial arrangement with respect to the inner member 202 by being positioned around the inner member 202 and displaced along an axis from the inner member 202. More specifically, in the example embodiment illustrated in Fig. 2, the inner member 202 is positioned partially inside the outer member 206 and thus protrudes from the outer member 206. However, it will be appreciated that in other embodiments, the outer member 206 may be positioned in a different spatial arrangement with respect to the inner member 202.

As mentioned earlier, the device 200 comprises at least one light source 208 arranged on at least one of the inner member 202 and the outer member 206. More specifically, in the illustrated example embodiment of Fig. 2, a light source 208 is arranged on the inner member 102. The at least one light source 208 is configured to emit light (not illustrated in Fig. 2) to illuminate the finger of the subject. Although only one light source 208 is illustrated in the example embodiment of Fig. 2, it will be understood that the device 200 may alternatively comprise a plurality of light sources 208.

As mentioned earlier, the device 200 described herein comprises at least one light detector 210 arranged on at least one of the inner member 202 and the outer member 206. In the illustrated example embodiment of Fig. 2, the at least one light detector 210 is arranged on the inner member 202. The at least one light detector 210 is configured to obtain optical signals from the finger of the subject. Although only one light detector 210 is illustrated in the example embodiment of Fig. 2, it will be understood that the device 200 may alternatively comprise a plurality of light detectors 210.

As described earlier, the inner member 202 is movable relative to the outer member 206 to enable the at least one light detector 210 to obtain optical signals for different light paths through the finger of the subject for use in measuring blood pressure. As illustrated by the arrow 218, in some embodiments such as the example illustrated in Fig. 2, the inner member 202 can be movable relative to the outer member 206 by being rotatable relative to the outer member 206 according to some embodiments.

Also, as illustrated by arrows 224 and 226, in some embodiments such as the example illustrated in Fig. 2, the inner member 202 can be movable relative to the outer member 206 by being axially movable (i.e. moveable along an axis or translatable) relative to the outer member 206 according to some embodiments. More specifically, in the example embodiment illustrated in Fig. 2, the inner member 202 and the outer member 206 are axially movable (i.e. moveable along an axis or translatable) in order for the inner member 202 to be movable relative to the outer member 206. However, it will be appreciated that in other embodiments, only one of the inner member 202 and the outer member 206 may be axially movable (i.e. moveable along an axis or translatable).

Although an example of the movement of the inner member 202 relative to the outer member 206 has been provided with reference to the example embodiment illustrated in Fig. 2, it will be understood that the inner member 202 can be movable relative to the outer member 206 in alternative or additional ways in other embodiments. For example, in some embodiments, the inner member 102 may alternatively or additionally be rotationally movable relative to the outer member 106 and/or moveable in any other way.

Thus, it is possible to move the inner member 202 relative to the outer member 206 until an optimum arrangement of the inner member 202 with respect to the outer member 206 is reached, whereby the optical signals obtained in that optimum arrangement provide optimum blood pressure measurements. For example, an arrangement that provides optimum blood pressure measurements can be an arrangement in which the at least one light detector 210 can obtain an optical signal for a light path through the artery in the finger of the subject (not illustrated in Fig. 2) for use in measuring blood pressure. In this way, the reliability of blood pressure measurements acquired from obtained optical signal can be improved.

Although not illustrated in Fig. 2, in some embodiments, the outer member 206 can be separated from the inner member 202 by a fluid (e.g. liquid or gas) chamber as described earlier.

As illustrated in Fig. 2, in some embodiments, the device 200 may comprise or be coupled to a processor 222 such as that described earlier. However, it will be understood that in other embodiments with a processor 222, the processor 222 may be separate from (e.g. external to or remote from) the device 200. In embodiments with a processor 222, as described earlier, the at least one light detector 210 may communicate with the processor 222 via a wired connection 228 (as illustrated in Fig. 2) or wirelessly.

As illustrated in Fig. 2, in some embodiments, the device 200 may comprise a handle 230. In some embodiments, the handle may be used to move or more easily move (e.g. rotate as illustrated by the arrow 218 in Fig. 2) the inner member 202 relative to the outer member 206.

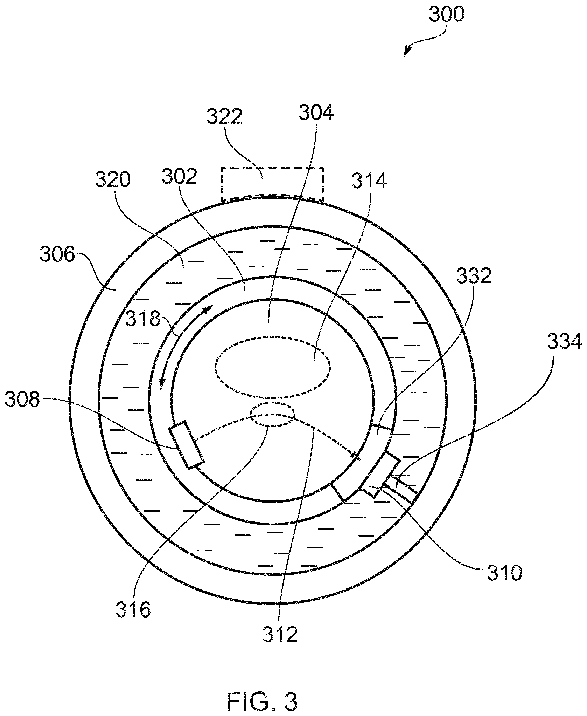

Fig. 3 illustrates an example of the device 300 for use in measuring blood pressure according to an embodiment. As illustrated in Fig. 3, the device 300 comprises an inner member 302 configured to be worn around a finger 304 of a subject. The device 300 also comprises an outer member 306 positioned in a spatial arrangement with respect to the inner member 302. As described earlier, the outer member 306 may be positioned in a spatial arrangement with respect to the inner member 302 by being positioned around the inner member 302 and/or displaced along an axis from the inner member 302.

As mentioned earlier, the device 300 comprises at least one light source 308 arranged on at least one of the inner member 302 and the outer member 306. More specifically, in the illustrated example embodiment of Fig. 3, the at least one light source 308 is arranged on the inner member 302. The at least one light source 308 is configured to emit light 312 to illuminate the finger 304 of the subject. The light 312 is illustrated in Fig. 3 with reference to a bone 314 of the finger 304 of the subject and an artery 316 in the finger 304 of the subject. Although only one light source 308 is illustrated in the example embodiment of Fig. 3, it will be understood that the device 300 may alternatively comprise a plurality of light sources 308.

As mentioned earlier, the device 300 described herein comprises at least one light detector 310 arranged on at least one of the inner member 302 and the outer member 306. In the illustrated example embodiment of Fig. 3, the at least one light detector 310 is arranged on the outer member 306. The at least one light detector 310 is configured to obtain optical signals from the finger 304 of the subject. Although only one light detector 310 is illustrated in the example embodiment of Fig. 3, it will be understood that the device 300 may alternatively comprise a plurality of light detectors 310.

As illustrated in Fig. 3 and as mentioned earlier, in some embodiments, the inner member 302 described herein can comprise a portion (e.g. an aperture or a transparent portion, such as a window) 332 through which at least one light source 308 is configured to emit light 312 to illuminate the finger 304 of the subject. In other words, in some embodiments, the inner member 302 described herein can comprise a portion (e.g. an aperture or a transparent portion, such as a window) 332 through which at least one light detector 310 is configured to obtain optical signals from the finger 304 of the subject. In the example embodiment illustrated in Fig. 3, the inner member 302 comprises a portion (e.g. an aperture or a transparent portion, such as a window) 332 through which at least one light source 308 arranged on the inner member 302 is configured to emit light 312 to illuminate the finger 304 of the subject. In other words, in the example embodiment illustrated in Fig. 3, the inner member 302 comprises a portion (e.g. an aperture or a transparent portion, such as a window) 332 through which at least one light detector 310 arranged on the outer member 306 is configured to obtain optical signals from the finger 304 of the subject. In some embodiments, the portion 332 may comprise the whole of the (i.e. the entire) inner member 302. In other embodiments, the portion 332 may comprise a part of the inner member 302. In some embodiments where the portion 332 comprises a part of the inner member 302, the at least one light detector 310 may be movable together with the portion 332 (or that part of the inner member 302).

In some embodiments, as illustrated in Fig. 3, at least one light detector 310 may be positioned on an extension member 334 that extends from the outer member 306 toward the inner member 302. The extension member 334 can support at least one light detector 310 according to some embodiments. For example, in some embodiments, the extension member 334 can be configured to fix at least one light detector 310 to the outer member 306. In this way, by moving the outer member 306, the at least one light detector 310 will also move. This makes it possible to change the distance between at least one light source 308 and at least one light detector 310.

As described earlier, the inner member 302 is movable relative to the outer member 306 to enable the at least one light detector 310 to obtain optical signals for different light paths through the finger 304 of the subject for use in measuring blood. As illustrated by the arrow 318, in some embodiments such as the example illustrated in Fig. 3, the inner member 302 can be movable relative to the outer member 306 by being rotatable relative to the outer member 306.

More specifically, in the example embodiment illustrated in Fig. 3, the inner member 302 is rotatable and the outer member 306 is fixed in order for the inner member 302 to be movable relative to the outer member 306. However, it will be appreciated that in other embodiments, the outer member 306 may be rotatable and the inner member 302 may be fixed or both the inner member 302 and the outer member 306 may be rotatable in order for the inner member 302 to be movable relative to the outer member 306. By way of the rotation of the inner member 302 relative to the outer member 306, a distance between the at least one light source 308 arranged on the inner member 302 and the at least one light detector 310 arranged on the outer member 306 can be varied.

Although an example of the movement of the inner member 302 relative to the outer member 306 has been provided with reference to the example embodiment illustrated in Fig. 3, it will be understood that the inner member 302 can be movable relative to the outer member 306 in alternative or additional ways in other embodiments. For example, in some embodiments, the inner member 302 may alternatively or additionally be axially movable relative to the outer member 306 and/or moveable in any other way.

Thus, it is possible to move the inner member 302 relative to the outer member 306 until an optimum arrangement of the inner member 302 with respect to the outer member 306 is reached, whereby the optical signals obtained in that optimum arrangement provide optimum blood pressure measurements. For example, an arrangement that provides optimum blood pressure measurements can be an arrangement in which the at least one light detector 310 can obtain an optical signal for a light path through the artery 316 in the finger 304 of the subject for use in measuring blood pressure. In this way, the reliability of blood pressure measurements acquired from obtained optical signal can be improved.

As illustrated in Fig. 3 and as described earlier, in some embodiments, the outer member 306 can be separated from the inner member 302 by a fluid (e.g. liquid or gas) chamber 320.

As also illustrated in Fig. 3, in some embodiments, the device 300 may comprise or be coupled to a processor 322 such as that described earlier. However, it will be understood that in other embodiments with a processor 322, the processor 322 may be separate from (e.g. external to or remote from) the device 300. In embodiments with a processor 322, as described earlier, the at least one light detector 310 may communicate with the processor 322 wirelessly or via a wired connection (not illustrated in Fig. 3).

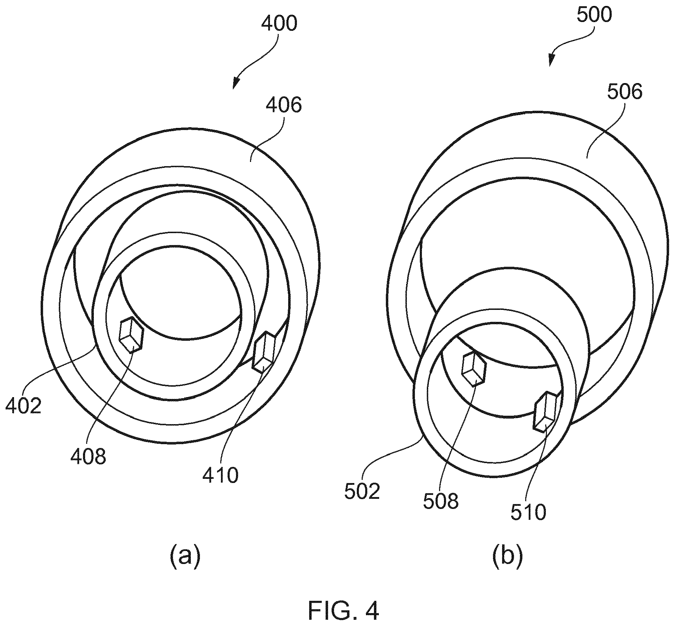

Figs. 4(a) and (b) illustrate examples of the device 400, 500 for use in measuring blood pressure according to other embodiments. As illustrated in Figs. 4(a) and (b), the device 400, 500 comprises an inner member 402, 502 configured to be worn around a finger of a subject (not illustrated in Figs. 4(a) and (b)). The device 400, 500 also comprises an outer member 406, 506 positioned in a spatial arrangement with respect to the inner member 402, 502. As described earlier, the outer member 406, 506 may be positioned in a spatial arrangement with respect to the inner member 502, 502 by being positioned around the inner member 402, 502 and/or displaced along an axis from the inner member 402, 502.

In the example embodiment illustrated in Fig. 4 (a), the outer member 406 is positioned in a spatial arrangement with respect to the inner member 402 by being positioned around the inner member 402. More specifically, the inner member 402 in the example embodiment illustrated in Fig. 4 (a) is positioned inside the outer member 406. In the example embodiment illustrated in Fig. 4 (b), the outer member 506 is positioned in a spatial arrangement with respect to the inner member 502 by being displaced along an axis from the inner member 506. More specifically, the inner member 502 in the example embodiment illustrated in Fig. 4 (b) is positioned adjacent to the outer member 506. However, it will be appreciated that in other embodiments, the outer member 406, 506 may be positioned in a different spatial arrangement with respect to the inner member 402, 502.

As mentioned earlier, the device 400, 500 comprises at least one light source 408, 508 arranged on at least one of the inner member 402 and the outer member 506. The at least one light source 408, 508 is configured to emit light (not illustrated in Fig. 4(a) and (b)) to illuminate the finger of the subject. In the illustrated example embodiment of Fig. 4(a), a light source 408 is arranged on the inner member 402. In the illustrated example embodiment of Fig. 4(b), a light source 508 is arranged on the outer member 506. Although only one light source is illustrated in the example embodiments of Figs. 4(a) and (b), it will be understood that the device 400, 500 may alternatively comprise a plurality of light sources 408, 508.

As mentioned earlier, the device 400, 500 described herein comprises at least one light detector 410, 510 arranged on at least one of the inner member 402, 502 and the outer member 406, 506. The at least one light detector 410, 510 is configured to obtain optical signals from the finger of the subject. In the illustrated example embodiment of Fig. 4(a), the at least one light detector 410 is arranged on the outer member 406. In the illustrated example embodiment of Fig. 4(b), the at least one light detector 510 is arranged on the inner member 502. Although only one light detector 410, 510 is illustrated in the example embodiments of Figs. 4 (a) and (b), it will be understood that the device 400, 500 may alternatively comprise a plurality of light detectors 410, 510.

As described earlier, the inner member 402, 502 is movable relative to the outer member 406, 506 to enable the at least one light detector 410, 510 to obtain optical signals for different light paths through the finger of the subject for use in measuring blood pressure. The inner member 402, 502 can be movable relative to the outer member 406, 506 in any of the ways described earlier and, although examples of the movement have been provided, it will be understood that the inner member 402, 502 can be movable relative to the outer member 406, 506 in alternative or additional ways.

Thus, it is possible to move the inner member 402, 502 relative to the outer member 406, 506 until an optimum arrangement of the inner member 402, 502 with respect to the outer member 406, 506 is reached, whereby the optical signals obtained in that optimum arrangement provide optimum blood pressure measurements. For example, an arrangement that provides optimum blood pressure measurements can be an arrangement in which the at least one light detector 410, 510 can obtain an optical signal for a light path through the artery in the finger of the subject (not illustrated in Figs. 4(a) and (b)) for use in measuring blood pressure. In this way, the reliability of blood pressure measurements acquired from obtained optical signal can be improved.

Although not illustrated in Figs. 4(a) and (b), in some embodiments, the outer member 406, 506 can be separated from the inner member 402, 502 by a fluid (e.g. liquid or gas) chamber as described earlier.

Although also not illustrated in Figs. 4(a) and (b), in some embodiments, the device 400, 500 may comprise or be coupled to a processor such as that described earlier. However, it will be understood that in other embodiments with a processor, the processor may be separate from (e.g. external to or remote from) the device 400, 500. In embodiments with a processor, as described earlier, the at least one light detector 410, 510 may communicate with the processor via a wired connection or wirelessly.

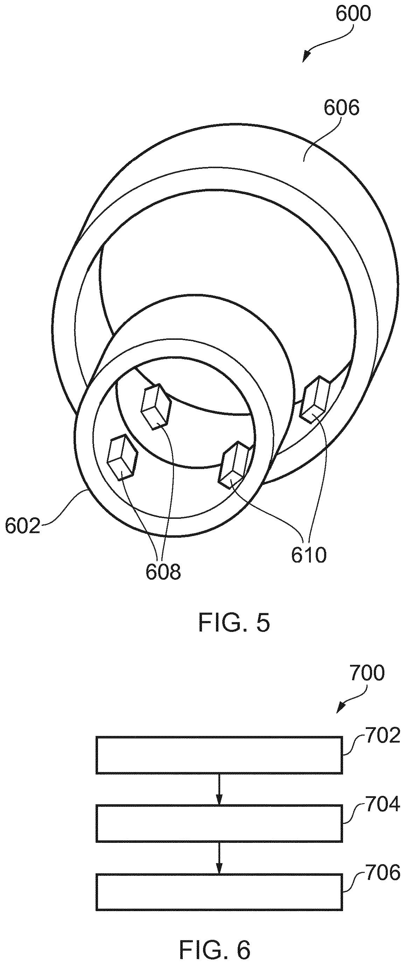

Fig. 5 illustrates an example of the device 600 for use in measuring blood pressure according to another embodiment. As illustrated in Fig. 5, the device 600 comprises an inner member 602 configured to be worn around a finger of a subject (not illustrated in Fig. 6). The device 600 also comprises an outer member 606 positioned in a spatial arrangement with respect to the inner member 602. As described earlier, the outer member 606 may be positioned in a spatial arrangement with respect to the inner member 602 by being positioned around the inner member 602 and/or displaced along an axis from the inner member 602. In the example embodiment illustrated in Fig. 5, the outer member 606 is positioned in a spatial arrangement with respect to the inner member 602 by being displaced along an axis from the inner member 602. More specifically, the inner member 602 in the example embodiment illustrated in Fig. 5 is positioned adjacent to the outer member 606. However, it will be appreciated that in other embodiments, the outer member 606 may be positioned in a different spatial arrangement with respect to the inner member 602.

As mentioned earlier, the device 600 comprises at least one light source 608 arranged on at least one of the inner member 602 and the outer member 606. The at least one light source 608, is configured to emit light (not illustrated in Fig. 6) to illuminate the finger of the subject. In the illustrated example embodiment of Fig. 6, a light source 608 is arranged on the inner member 602 and the outer member 606. Although two light sources 608 are illustrated in the example embodiment of Fig. 6, it will be understood that the device 600 may alternatively comprise at least one further light source 608 arranged on any one or more of the inner member 602 and the outer member 606 or only a single light source 608 arranged on either the inner member 602 or the outer member 606.

As mentioned earlier, the device 600 described herein comprises at least one light detector 610 arranged on at least one of the inner member 602 and the outer member 606. The at least one light detector 610 is configured to obtain optical signals from the finger of the subject. In the illustrated example embodiment of Fig. 6, a light detector 610 is arranged on the inner member 602 and the outer member 606. Thus, in the illustrated example embodiment of Fig. 6, each of the inner member and the outer member have at least one light source 608 and at least one light detector 610 arranged thereon (e.g. each of the inner member and the outer member may have a unit arranged thereon, wherein the unit comprises at least one light source 608 and at least one light detector 610). Although two light detectors 610 are illustrated in the example embodiment of Fig. 6, it will be understood that the device 600 may alternatively comprise at least one further light detector 610 arranged on any one or more of the inner member 602 and the outer member 606 or only a single light detector 610 arranged on either the inner member 602 or the outer member 606.

As described earlier, the inner member 602 is movable relative to the outer member 606 to enable the at least one light detector 610 to obtain optical signals for different light paths through the finger of the subject for use in measuring blood pressure. The inner member 602 can be movable relative to the outer member 606 in any of the ways described earlier and, although examples of the movement have been provided, it will be understood that the inner member 602 can be movable relative to the outer member 606 in alternative or additional ways.

Thus, it is possible to move the inner member 602 relative to the outer member 606 until an optimum arrangement of the inner member 602 with respect to the outer member 606 is reached, whereby the optical signals obtained in that optimum arrangement provide optimum blood pressure measurements. For example, an arrangement that provides optimum blood pressure measurements can be an arrangement in which the at least one light detector 610 can obtain an optical signal for a light path through the artery in the finger of the subject (not illustrated in Fig. 6) for use in measuring blood pressure. In this way, the reliability of blood pressure measurements acquired from obtained optical signal can be improved.

Although not illustrated in Fig. 6, in some embodiments, the outer member 606 can be separated from the inner member 602 by a fluid (e.g. liquid or gas) chamber as described earlier.

Although also not illustrated in Fig. 6, in some embodiments, the device 600 may comprise or be coupled to a processor such as that described earlier. However, it will be understood that in other embodiments with a processor, the processor may be separate from (e.g. external to or remote from) the device 600. In embodiments with a processor, as described earlier, the at least one light detector 610 may communicate with the processor via a wired connection or wirelessly.

Although not illustrated in the figures, the device 100, 200, 300, 400, 500, 600 described herein may comprise a battery or other power supply for powering the device 100, 200, 300, 400, 500, 600 or means for connecting the device 100, 200, 300, 400, 500, 600 described herein to a mains power supply. It will also be understood that the device 100, 200, 300, 400, 500, 600 described herein may comprise any other component to those described herein or any combination of components.

In some embodiments, the inner member 102, 202, 302, 402, 502, 602 and the outer member 106, 206, 306, 406, 506, 606 of the device 100, 200, 300, 400, 500, 600 described herein can be detachably coupled to each other. In other words, in some embodiments, the inner member 102, 202, 302, 402, 502, 602 and the outer member 106, 206, 306, 406, 506, 606 can be detached from each other. In some of these embodiments, a battery may be arranged on the outer member 106, 206, 306, 406, 506, 606. In this way, the outer member 106, 206, 306, 406, 506, 606 can be detached from the inner member 102, 202, 302, 402, 502, 602 for the battery arranged on the outer member 106, 206, 306, 406, 506, 606 to be charged (e.g. on a docking station that is separate or remote from the device 100, 200, 300, 400, 500, 600). In some of these embodiments, another outer member may replace the detached outer member 106, 206, 306, 406, 506, 606, e.g. while the battery is charged.

It will be understood that any one or more of the features of described herein can be combined with any one or more of the other features described herein. For example, any one or more of the features of an example embodiment of the device 100, 200, 300, 400, 500, 600 described earlier can be combined with any one or more of the features from any other example embodiment of the device 100, 200, 300, 400, 500, 600 described earlier.

Fig. 6 illustrates a method 700 of operating the device 100, 200, 300, 400, 500, 600 described herein for use in measuring blood pressure according to an embodiment. The method 700 can generally be performed by or under the control of the processor 122, 222, 322 described earlier.

With reference to Fig. 6, at block 702, the light to illuminate the finger 104, 304 of the subject is emitted. More specifically, the at least one light source 108, 208, 308, 408, 508, 608 emits the light to illuminate the finger 104, 304 of the subject. In some embodiments, the processor 122, 222, 322 can be configured to control the at least one light source 108, 208, 308, 408, 508, 608 to emit the light to illuminate the finger 104, 304 of the subject.

At block 704, the inner member 102, 202, 302, 402, 502, 602 is moved relative to the outer member 106, 206, 306, 406, 506, 606. In some embodiments, the processor 122, 222, 322 can be configured to control the movement of the inner member 102, 202, 302, 402, 502, 602 relative to the outer member 106, 206, 306, 406, 506, 606. Alternatively or in addition, in other embodiments, the inner member 102, 202, 302, 402, 502, 602 may be manually moved relative to the outer member 106, 206, 306, 406, 506, 606.

At block 706, the optical signals for the different light paths through the finger 104, 304 of the subject are obtained from the finger 104, 304 of the subject for use in measuring blood pressure. More specifically, the at least one detector 110, 210, 310, 410, 510, 610 obtains, from the finger 104, 304 of the subject, the optical signals for the different light paths through the finger 104, 304 of the subject for use in measuring blood pressure. In some embodiments, the processor 122, 222, 322 can be configured to control the at least one detector 110, 210, 310, 410, 510, 610 to obtain, from the finger 104, 304 of the subject, the optical signals for the different light paths through the finger 104, 304 of the subject for use in measuring blood pressure.

Although not illustrated in Fig. 6, in some embodiments, the method may further comprise processing the obtained optical signals for the different light paths through the finger 104, 304 of the subject to select a position for the inner member 102, 202, 302, 402, 502, 602 relative to the outer member 106, 206, 306, 406, 506, 606 for use in measuring a blood pressure of the subject. More specifically, the processor 122, 222, 322 processes the obtained optical signals for the different light paths through the finger 104, 304 of the subject to select a position for the inner member 102, 202, 302, 402, 502, 602 relative to the outer member 106, 206, 306, 406, 506, 606 for use in measuring a blood pressure of the subject according to some embodiments.

There is also provided a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein a device, a system, a method and a computer program product that address the limitations associated with the existing techniques. Any one or more of the embodiments described herein may be combined.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A device (100, 200, 300, 400, 500, 600) for use in measuring blood pressure, wherein the device (100, 200, 300, 400, 500, 600) comprises:
an inner member (102, 202, 302, 402, 502, 602) configured to be worn around a finger (104, 304) of a subject;
an outer member (106, 206, 306, 406, 506, 606) positioned in a spatial arrangement with respect to the inner member (102, 202, 302, 402, 502, 602);
at least one light source (108, 208, 308, 408, 508, 608) arranged on at least one of the inner member (102, 202, 302, 402, 502, 602) and the outer member (106, 206, 306, 406, 506, 606), the at least one light source (108, 208, 308, 408, 508, 608) configured to emit light (112, 312) to illuminate the finger (104, 304) of the subject; and
at least one light detector (110, 210, 310, 410, 510, 610) arranged on at least one of the inner member (102, 202, 302, 402, 502, 602) and the outer member (106, 206, 306, 406, 506, 606), the at least one light detector (110, 210, 310, 410, 510, 610) configured to obtain optical signals from the finger (104, 304) of the subject,
wherein the inner member (102, 202 302, 402, 502, 602) is movable relative to the outer member (106, 206, 306, 406, 506, 606) to enable the at least one light detector (110, 210, 310, 410, 510, 610) to obtain optical signals for different light paths through the finger (104, 304) of the subject for use in measuring blood pressure.

2. The device (100, 200, 300) as claimed in claim 1, wherein the inner member (102, 202, 302) is movable relative to the outer member (106, 206, 306) by being any one or more of:
rotatably (118, 218, 318) movable relative to the outer member (106, 206, 306); and
axially (224, 226) movable relative to the outer member (206).

3. The device (100, 200, 300, 400, 500, 600) as claimed in any of the preceding claims, wherein the outer member (106, 206, 306, 406, 506, 606) is positioned in a spatial arrangement with respect to the inner member (102, 202, 302, 402, 502, 602) by being any one or more of:
positioned around the inner member (102, 202, 302, 402); and displaced along an axis from the inner member (202, 502, 602).

4. The device (100, 200, 300, 400, 500, 600) as claimed in any of the preceding claims, wherein the outer member (106, 206, 306, 406, 506, 606) is mechanically independent of the inner member (102, 202, 302, 402, 502, 602).

5. The device (100, 300) as claimed in any of the preceding claims, wherein the outer member (106, 306) is separated from the inner member (102, 302) by a fluid chamber (120, 320).

6. The device (100, 300) as claimed in claim 5, wherein the fluid chamber (120, 320) is configured to hold a pressurized fluid.

7. The device (100, 200, 300, 400, 500, 600) as claimed in any of the preceding claims, wherein the inner member (102, 202, 302, 402, 502, 602) is flexible and/or the outer member (106, 206, 306, 406, 506, 606) is rigid.

8. The device (100, 200, 300, 400, 500, 600) as claimed in any of the preceding claims, wherein the inner member (102, 202, 302, 402, 502, 602) and the outer member (106) are arranged such that, in use, only the inner member (102, 202, 302, 402, 502, 602) is in contact with the finger (104, 304) of the subject.

9. The device (300) as claimed in any of the preceding claims, wherein the inner member (302) comprises a portion (332) through which any one or more of:
at least one light source, when arranged on the outer member, is configured to emit light to illuminate the finger of the subject; and
at least one light detector (310), when arranged on the outer member (306), is configured to obtain optical signals from the finger (304) of the subject.

10. The device (100, 200, 300, 400, 500, 600) as claimed in any of the preceding claims, wherein a distance between the at least one light source (108, 208, 308, 408, 508, 608) and the at least one light detector (110, 210, 310, 410, 510, 610) is variable.

11. A system for use in measuring blood pressure, the system comprising:
the device (100, 200, 300) as claimed in any of the preceding claims; and
a processor (122, 222, 322) configured to:
process the obtained optical signals for the different light paths through the finger (104, 304) of the subject to select a position for the inner member (102, 202, 302) relative to the outer member (106, 206, 306) for use in measuring a blood pressure of the subject.

12. The system as claimed in claim 11, wherein the processor (122, 222, 322) is configured to:
select the position for the inner member (102, 202, 302) relative to the outer member (106, 206, 306) for use in measuring the blood pressure of the subject based on an amplitude of the obtained optical signals for the different light paths through the finger (104, 304) of the subject.

13. A method of operating a device (100, 200, 300, 400, 500, 600) as claimed in any of claims 1 to 10, the method comprising:
emitting the light (112, 312) to illuminate the finger (104, 304) of the subject;
moving the inner member (102, 202, 302, 402, 502, 602) relative to the outer member (106, 206, 306, 406, 506, 606); and
obtaining, from the finger (104, 304) of the subject, the optical signals for the different light paths through the finger (104, 304) of the subject for use in measuring blood pressure.

14. The method as claimed in claim 13, further comprising:
processing the obtained optical signals for the different light paths through the finger (104, 304) of the subject to select a position for the inner member (102, 202, 302, 402, 502, 602) relative to the outer member (106, 206, 306, 406, 506, 606) for use in measuring a blood pressure of the subject.

15. A computer program product comprising a computer readable medium, the computer readable medium having a computer readable code embodied therein, the computer readable code being configured such that, on execution by a suitable computer or processor, the computer or processor is caused to perform the method as claimed in claim 13 or 14.
